Europäisches Patentamt

European Patent Office

Office européen des brevets

(11) Publication number: **0 458 339 A1**

# EUROPEAN PATENT APPLICATION

(21) Application number: 91108371.5

(22) Date of filing: 23.05.91

(51) Int. Cl.5: **A61B 5/14**, A61M 1/00

(30) Priority: 24.05.90 US 528801

(43) Date of publication of application:
27.11.91 Bulletin 91/48

(84) Designated Contracting States:
CH DE FR GB IT LI

(71) Applicant: SPACELABS, INC.
4200- 150th Avenue N.E.
Redmond Washington 98073-9713(US)

(72) Inventor: Schlosser, Mark S.
3036 47th S.W.
Seattle, Washington 98116(US)

(74) Representative: Patentanwälte Grünecker,
Kinkeldey, Stockmair & Partner
Maximilianstrasse 58
W-8000 München 22(DE)

(54) **Disposable blood sampling cartridge.**

(57) A blood sampling device having a housing (10), a needle unit (42) attached to the housing (10) for withdrawing blood from the patient into a blood reservoir (22) in the housing (10), a piston unit (18) disposed in the house (10) for pumping the blood from the blood reservoir (22) into a flow cell (11) and a plurality of ampules (16) containing analyzing fluids disposed in the housing (10) for flushing the fluid through the analyzer (11) during the analyzing procedure. The housing (10) includes a front portion (12) and a rear portion (14) which are rotatably disposed with respect to one another, the rear face (13) of the front portion (12) mating with the front face (15) of the rear portion (14). The front portion (12) has a blood reservoir (22), a vent line (28), inlet and outlet ports (32,30) and a waste chamber (26). The rear portion (14) includes a groove in the front face (15) thereof which, depending on the angular orientation of the rear portion (14) with respect to the front portion (12), connects the blood reservoir (22) to either the vent line (28) or the outlet port (30). Additionally, the rear portion (14) has a plurality of ampule receiving bores (62) for storing the ampules (16). Correspondingly, the rear portion (14) includes a plurality of fluid ports for respectively communicating the openings of ampules (16) to the outlet port (30) depending on the angular position of the rear housing portion (14) with respect to the front housing portion (12).

FIG.1

FIG.2

Technical Field

This invention relates to a device for sampling blood from arterial sources including a storage and valving system for transferring the blood sample and various fluids to an external analyzer for analyzing the sample.

Background of the Invention

Conventionally, blood analyzing systems include a syringe into which the blood is collected and an analyzer which analyzes the blood for such parameters as chemical composition, blood gas constituents, pH, etc. Two types of syringes have been used to withdraw the blood from the patient's arteries. The first type includes a vented piston arrangement wherein a needle is inserted in a patient's artery, and the blood is forced into the syringe by the pressure differential between the absolute pressure in the artery and atmospheric pressure. In this first type, the air in the syringe is vented to the atmosphere. The second type of syringe includes a piston which is moved to increase the volume in the syringe and thereby create a vacuum to suction the blood from the patient.

Once the blood has been withdrawn from the patient into a syringe, the syringe is delivered to a lab for analysis. The sample blood is then introduced into a conventional blood analyzer. Conventional blood analyzers include a plurality of containers for respective calibrating or analyzing fluids, and a waste container for storing the spent fluids and blood.

The problem with this arrangement is that conventional analyzers are too large and expensive to be located at a patient's bedside. Accordingly, the blood sample must be sent to the lab for analysis thereby requiring a relatively long period of time to obtain results. Additionally, conventional analyzers are relatively complex to operate, making bedside use impractical. For instance, the technician must insure that the analyzing fluids are not depleted.

Efforts have been made to simplify and miniaturize conventional analyzers by providing conventional analyzers with a disposable cartridge. The cartridge maintains the level of the analyzing fluids in the analyzer and provides a waste container for the spent analyzing fluids and blood. While such an arrangement does simplify the analyzing procedure, the disposable cartridge is extremely expensive, making the device commercially impractical. Moreover, as with the conventional analyzer described above, a pumping system is required in the analyzer to pump the blood and analyzing fluids through the device, thereby adding both size and weight to the analyzer.

Summary of the Invention

The present invention resides in a blood sampling cartridge, comprising a housing, a needle unit attached to the housing for withdrawing blood from the patient into a blood reservoir in the housing, a piston unit disposed in the housing for pumping the blood from the blood reservoir into a flow cell, a plurality of ampules containing analyzing fluids disposed in the housing for flushing the fluid through the analyzer during the analyzing procedure, and a waste chamber disposed in the housing for storing the spent fluids. The housing includes a front portion and a rear portion which are rotatably disposed with respect to one another, the rear face of the front portion mating with the front face of the rear portion. The front portion has a blood reservoir, a vent line, cartridge inlet and outlet ports and the waste chamber; the blood reservoir, vent line and cartridge outlet port communicating with the rear face of the front portion. The rear portion includes a linear trench-like groove in the front face thereof which, depending on the angular orientation of the rear portion with respect to the front portion, connects the blood reservoir to either the vent line or the cartridge outlet port.

Additionally, the rear portion has a plurality of ampule receiving bores for storing the ampules and a plurality of fluid ports for respectively communicating the openings of ampules to the cartridge outlet port depending on the angular position of the rear housing portion with respect to the front housing portion.

Initially, the rear portion of the housing is positioned such that the blood reservoir communicates with the vent line permitting the blood to be withdrawn from the patient into the blood reservoir. At this stage the flow cell ports are covered. Then the rear portion is rotated to align the blood reservoir with the cartridge outlet port such that the piston unit can pump the blood into the flow cell. The flow cell ports are uncovered to allow access to the flow cell connectors. Thereafter, the rear portion is successively rotated to successively connect each of the ampules to the cartridge outlet port such that the fluid contained in each of the ampules can be successively pumped from each of the ampules into the flow cell.

Brief Description of the Drawings

Figure 1 is an isometric view of the disposable blood sampling cartridge according to the present invention shown in the 0° position.

Figure 2 is a cross-sectional view of the disposable cartridge taken along line 2-2 of Figure 1.

Figure 3 is a schematic, cross-sectional view of the disposable cartridge taken along line 3-3 of Figure 2.

Figure 4 is a cross-sectional view of the disposable cartridge in the 60° position taken along line 4-4 of Figure 1.

Figure 5 is a schematic, cross-sectional view of the disposable cartridge taken along line 5-5 of Figure 4.

Figure 6 is a cross-sectional view of the disposable cartridge in the 120° position taken along line 6-6 of Figure 1.

Figure 7 is a schematic cross-sectional view of the disposable cartridge taken along line 7-7 of Figure 6.

Figure 8 is a schematic, cross-sectional view of the disposable cartridge in the 180° position taken along line 8-8 of Figure 1.

Figure 9 is a schematic, cross-sectional view of the disposable cartridge in the 240° taken along line 9-9 of Figure 1.

Figure 10 is a schematic, cross-sectional view of the disposable cartridge in the 300° position taken along line 10-10 of Figure 1.

Detailed Description of the Invention

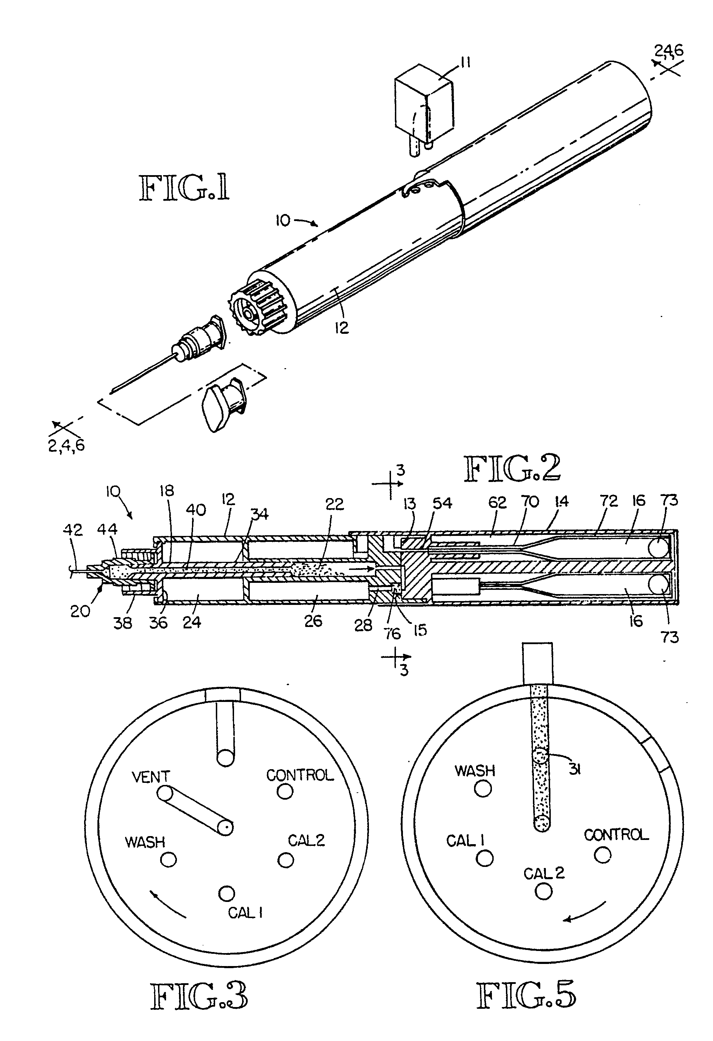

As shown in Figure 1, the present invention is embodied in a blood sampling cartridge 10 including front 12 and rear 14 cylindrical portions. A needle 42 projects from the front portion 12, and the front and rear portions 12, 14, respectively, are rotatable with respect to each other. A cap 45 is also shown for covering a blood inlet 40 of the front portion after the blood has been withdrawn and the needle removed. As explained below, rotation of the front and rear portions 12, 14 with respect to each other between each of several discrete positions controls:

    (a) the flow of blood

        (i) through the needle 42 into the front portion 12;

        (ii) from the front portion 12 to a flow cell 11 through an outlet port 30; and

    (b) the flow of spent blood, calibrating, control and washing fluids from respective reservoirs in the rear portion 14 to the flow cell 11 through the outlet port 30 and into the waste chamber in the front portion 12.

The flow cell 11 is of conventional design and, taken alone and apart from the blood sampling device 10, is not considered to be inventive.

Referring also now to Figure 2, the front portion 12 includes a blood reservoir 22 connected to the needle through the blood inlet 40, a piston receiving portion 24, an annular waste chamber 26, a vent line 28 communicating with the waste chamber, and cartridge outlet and inlet ports 30, 32, respectively. The ports 30, 32 are normally closed by a cover 100 that projects forwardly from the rear portion 14. The blood reservoir 22, vent line 28 and cartridge outlet port 30 each have a longitudinal portion which communicates with a rear face 13 of the front portion 12. In particular, the blood reservoir 22 is disposed directly on the longitudinal axis of the front portion 12 while the vent line 28 and cartridge outlet port 30 are radially displaced from the longitudinal axis by an equal distance. The needle unit 20 includes a needle 42 as well as a needle attaching portion 44. The needle attaching portion is designed to be slid onto one end of the tubular portion 34 of the piston unit 18 such that the drive hex 38 circumscribes the needle attaching portion in the manner illustrated in Figure 2.

Referring also to Figure 3, the vent line 28 has a vent 76 disposed therein which permits air to pass therethrough while preventing the passage of blood. Natural venting is provided through the cartridge inlet port 32 when blood is withdrawn and through the vent line 28 when spent fluids are introduced into the waste chamber 26.

The rear portion 14 is also cylindrical in shape and has a diameter slightly larger than the diameter of the front portion 12. In this manner, a front end of the rear portion 14 is rotatably disposed over the rear end of the front portion 12 such that the front face 15 of the rear portion abuts against the rear face 13 of the front portion 12, as illustrated in Figure 2. A groove 74 is provided on the front face 15 of the rear portion. The groove extends radially from the center of the face, terminating short of the outer circumference of the rear portion 14. The groove is designed to interconnect the blood reservoir 22 of the front portion 12 to either the vent line 28 (Figures 2 and 3) or the cartridge outlet port 30 by rotating the front portion 12 relative to the rear portion 14, as will be described in detail below.

The piston unit 18 includes axially extending tubular portion 34, flange 36 and drive hex 38, as illustrated in Figure 2. The tubular portion 34 defines the blood inlet 40 which communicates with the blood reservoir 22 of the front portion 12. The tubular portion 34 is telescopically disposed in the bore forming the blood reservoir while the flange 36 is slidably disposed in the piston receiving portion 24 of the front portion. The piston unit 18 is actuated to expel blood from the blood reservoir 22, as described in detail below.

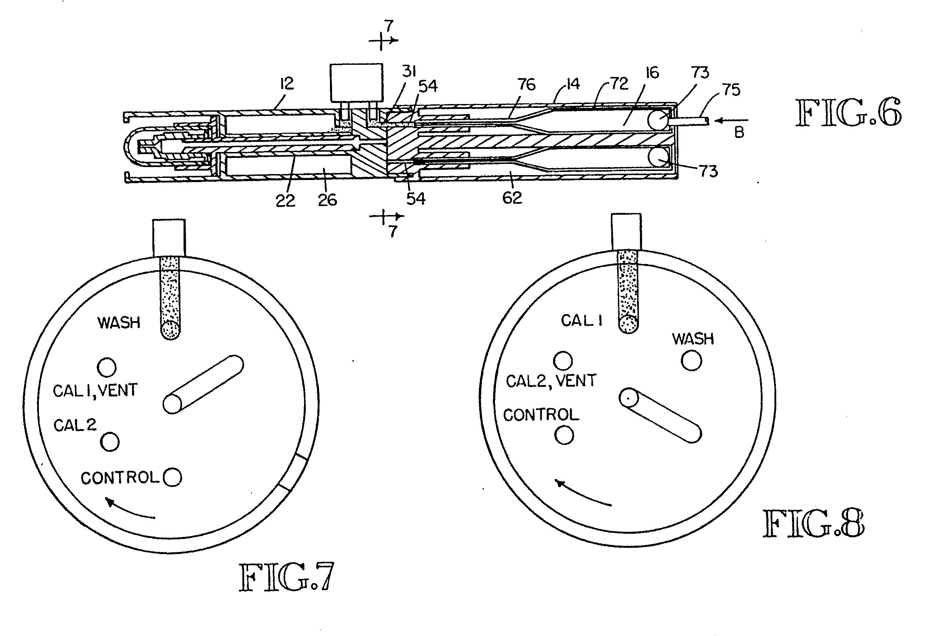

As illustrated in Figure 6, the cartridge outlet port 30 is substantially L-shaped having the above-described longitudinal portion 31 as well as a radially extending portion 33 which extends to the outer circumference of the front portion. The cartridge inlet port 32 also extends in the radial direction and communicates with the waste chamber 26. The inlet port 32 is disposed adjacent the outlet port 30 allowing convenient access of the flow cell to the ports.

With reference to Figures 2 and 3, the rear portion 14 includes four axially extending ampule ports 54 and a corresponding number of ampule receiving bores 62. Each of the ampules 16 includes a small diameter stem portion 70 and a large diameter base portion 72. The ampules are respectively disposed in the ampule receiving bores by respectively inserting the small diameter portions in the ampule ports. Each of the ampules contains a different analyzing fluid: wash, calibrant 1, calibrant 2, and control. The analyzing fluids are utilized to wash, calibrate, and flush the flow cell 11 during the blood analysis. The ampule ports 54 communicate with the front face 15 of the rear portion 14 and are sequentially aligned with the longitudinally disposed portion 31 (Figure 6) of the cartridge outlet port 30 such that the ampule fluids can be individually introduced into the flow cell 11 by successively rotating the front portion with respect to the rear portion of the device a predetermined number of degrees.

Each of the ampules 16 has a ball 73 which acts as a piston to pump the fluid therefrom. Specifically, as illustrated in Figure 6, the rear of each ampule is fractured using push rod 75 enabling the push rod to slide the ball in the direction of the ampule stem 70, as illustrated by arrow B. In this manner the fluids are pumped from the ampules into the flow cell via the ampule ports 54 and the outlet port 30.

The volume of the blood reservoir is relatively small so as to minimize the amount of blood withdrawn from the patient. Correspondingly, the passages in the cartridge are designed to be relatively short in length and small in diameter to insure that a minimal amount of blood is lost when the blood flows into the flow cell so that there is a sufficient amount of blood in the flow cell for analysis. Additionally, the front portion 12 may be manufactured out of a transparent material to permit the user to view the amount of blood in the reservoir. Finally, an absorbent material may be packed in the waste chamber to absorb the spent fluids.

The operation of the device is as follows. Initially, the front portion 12 and rear portion 14 are rotatably aligned in the manner illustrated in Figures 1, 2 and 3 with the cover 100 blocking the flow cell ports 30, 32. For explanation, this position will be referred to as the 0° position. In the 0° position, blood is withdrawn from the patient's artery, relying on the arterial pressure to force the blood into the device. As illustrated in Figures 2 and 3, the blood reservoir 22 of the front portion 12 is connected to the vent line 28 via the groove 74 for permitting the air in the blood inlet to be vented through the waste chamber 26 so as to allow the blood to enter the device. Once the flow of blood reaches the vent 76 provided at the inlet to the vent line 28 the flow of blood from the patient's artery stops.

Thereafter, the needle is withdrawn from the patient, removed from the device and a luer lock cover 45 (Figure 4) secured to the front end of the piston unit 18. The device is then placed in an analyzer having a flow cell 11 (Figure 1). The analyzer rotates the rear portion 14 of the device approximately 40° with respect to the front portion 12 to expose the flow cell ports 30, 32. At this position, the flow cell 11 in the analyzer is mated to the outlet and inlet ports 30, 32, respectively. Thereafter, the rear portion is rotated an additional 20° to the 60° position to align the groove 74 with the longitudinal portion 31 of outlet port 30, as illustrated in Figures 4 and 5. In this position, the blood reservoir 22 communicates with the outlet port 30 through the groove 74, longitudinal portion 31 and radially extending portion 33. At this time, the analyzer slides the piston unit 18 in the direction of arrow A of Figure 4 a predetermined distance causing the blood in the blood reservoir 22 to be introduced into the flow cell. Thereafter, the blood is analyzed.

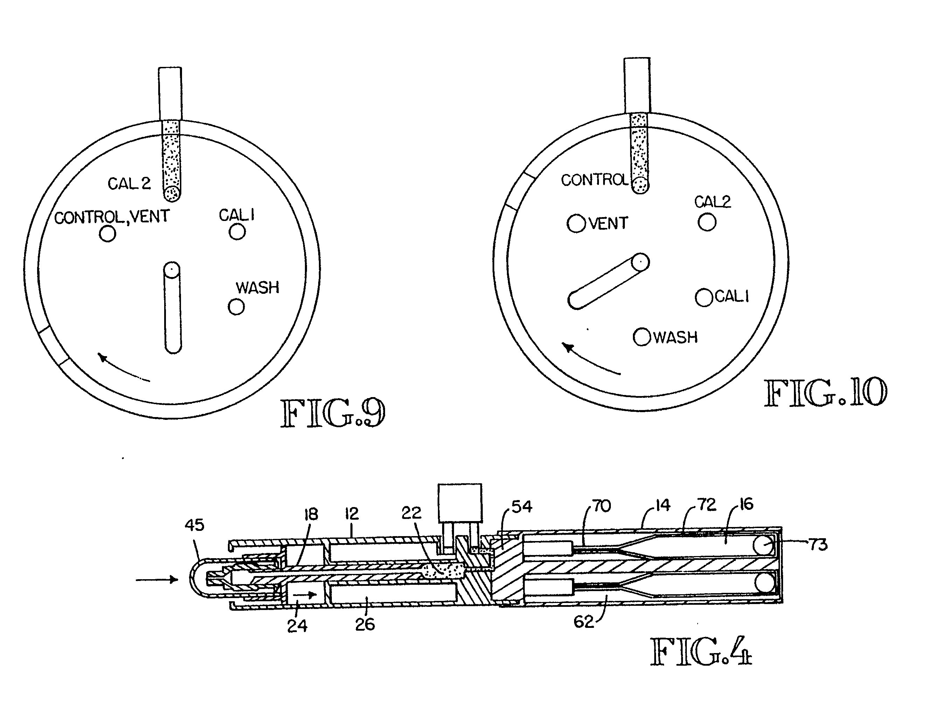

After the blood has been analyzed, the analyzer rotates another 60° to the 120° position, as illustrated in Figures 6 and 7. In this position, the ampule port 54 for the wash is aligned with the longitudinal portion 31 of the cartridge outlet port 30. At this time, a mechanism in the analyzer fractures the stem 70, the push rod 75 in the analyzer fractures the base of the ampule and, thereafter, the push rod 75 pushes the piston ball 73 in the direction illustrated by arrow B in Figure 6 forcing the wash fluid from the ampule and into the flow cell 11. The wash fluid thereby flushes the blood from the flow cell through the cartridge inlet port 32 and into the annular waste chamber 26. Thereafter, the rear portion 14 is rotated another 60° to the 180° position, illustrated in Figure 8, aligning the calibrant 1 ampule port 54 with the longitudinal portion 31 of cartridge outlet port 30. Again, the ampule stem and base are fractured and the calibrant 1 fluid forced into the flow cell to calibrate the analyzer and flush the wash fluid from the flow cell and into the waste chamber 26. These steps are repeated for the calibrant 2 ampule at the 240° position (Figure 9) and the control ampule at the 300° position (Figure 10). Finally, after the control fluid has been introduced into the flow cell 11, the analyzer rotates the rear portion another 20° to the 320° position and detaches the cartridge from the flow cell. Thereafter, the analyzer rotates the rear portion another 40° to the initial 0° position such that the cover 76 of the rear portion 14 closes the flow cell ports 30, 32, as illustrated in Figures 1 and 3. The device can then be disposed of without the user ever contacting the blood or any of the fluids.

While the preferred embodiment of the invention has been described with the disposable cartridge storing four ampules, it is understood that the cartridge could store fewer or more ampules as required. Moreover, while the preferred embodiment describes the manner in which the cartridge is connected to a flow cell, it is understood that the cartridge could be connected to any appropriate analyzing device. Furthermore, while the invention has been described with reference to withdrawing blood from an artery and the subsequent analysis of the blood, it is understood that the device could be used to withdraw and analyze any fluid from a pressurized source.

## Claims

1. A device for withdrawing blood from a patient and for passing the blood through a flow cell for analysis, comprising:

   a housing, said housing including a front portion and a rear portion rotatably disposed with respect to one another, one of said portions having a cartridge outlet port, said front portion having a blood reservoir disposed therein, said rear portion having connecting means for connecting said blood reservoir and said outlet port based on the angular orientation of said front portion with respect to said rear portion;

   means for introducing blood into said blood reservoir; and

   means for transferring said blood from said blood reservoir to said flow cell via said outlet port.

2. The device of claim 1 wherein said front portion further includes a vent line disposed therein for venting air from said blood reservoir when said blood is introduced therein, and wherein said connecting means connects said blood reservoir to one of said outlet port and said vent line depending on the angular orientation of said front portion with respect to said rear portion.

3. The device of claim 1 wherein said front portion further includes a cartridge inlet port for receiving said blood from said flow cell after said blood has been analyzed.

4. The device of claim 3 wherein said front portion further includes a waste chamber disposed therein for storing said blood received by said inlet port.

5. The device of claim 4 wherein said waste chamber is annular in shape, circumscribing said blood reservoir.

6. The device of claim 1 wherein said transferring means comprises a piston slidably disposed in said blood reservoir for pumping said blood therefrom.

7. The device of claim 1 wherein said rear portion includes means for storing at least one ampule containing an analyzing fluid.

8. The device of claim 7, further comprising means for forcing said analyzing fluid from said ampule through an opening therein.

9. The device of claim 8 wherein said rear portion further includes a fluid port for communicating said opening in said ampule to said outlet port depending on the angular position of said rear portion with respect to said front portion.

10. The device of claim 3 wherein said front portion and said rear portion are mated together at a rear end face and a front end face, respectively.

11. The device of claim 10 wherein said blood reservoir, said vent line and said outlet port each include a portion that extends axially with respect to said front portion and opens into said rear end face of said front portion, said axially extending portion of said blood reservoir being disposed along the longitudinal axis of said front portion.

12. The device of claim 11 wherein each of said axially extending portions of said vent line and said outlet port are disposed at the same radial position from the longitudinal axis of said front portion.

13. The device of claim 12 wherein said connecting means is a groove disposed in said front face of said rear portion and extending in the radial direction from the center of said face to the same radial position as said axially extending portions of said vent line and outlet port such that said connecting means can connect said blood reservoir to one of said vent line and said outlet port by rotating said rear portion with respect to said front portion.

14. The device of claim 12 wherein said rear portion includes means for storing at least one ampule containing an analyzing fluid.

15. The device of claim 14, further comprising means for forcing said analyzing fluid from said ampule through an opening therein.

16. The device of claim 15 wherein said rear portion further includes a fluid port extending axially with respect to said rear portion and being located at the same radial position as said cartridge outlet port such that by rotating said rear portion with respect to said front portion said fluid port can be aligned with said outlet port so as to communicate said opening in said ampule with said outlet port.

FIG.1

FIG.2

FIG.3

VENT
CONTROL
WASH
CAL2
CAL1

FIG.5

WASH
CONTROL
CAL1
CAL2

7

FIG.6

FIG.7

FIG.8

EP 0 458 339 A1

CAL.2

CONTROL,VENT     CAL1

WASH

FIG.9

CONTROL

VENT     CAL2

CAL1

WASH

FIG.10

FIG.4

45    18    12    22    54    70    14    72    16    73

24    26    62

EP 0 458 339 A1

## DOCUMENTS CONSIDERED TO BE RELEVANT

EP 91108371.5

| Category | Citation of document with indication, where appropriate, of relevant passages | Relevant to claim | CLASSIFICATION OF THE APPLICATION (Int. Cl.5) |
|---|---|---|---|
| A | US - A - 3 776 218 (J.A. SVENSSON) * Totality * -- | 1 | A 61 B 5/14 A 61 M 1/00 |
| A | GB - A - 1 538 196 (W.W. FEASTER) * Totality * ---- | 1 | |

**TECHNICAL FIELDS SEARCHED (Int. Cl.5)**

A 61 B
A 61 M

The present search report has been drawn up for all claims

| Place of search | Date of completion of the search | Examiner |
|---|---|---|
| VIENNA | 04-09-1991 | LUDWIG |